# EUROPÄISCHE PATENTANMELDUNG

(11) **EP 4 620 413 A1**
(43) Veröffentlichungstag der Anmeldung: **24.09.2025**
(21) Anmeldenummer: 25164657.6
(22) Anmeldetag: 19.03.2025
(51) Int. Cl.: A61B 17/70

(54) **MEDIZINISCHES INSTRUMENT ZUR WIRBELMANIPULATION**

(30) Priorität: 22.03.2024 DE 102024108234
(71) Anmelder: Aesculap AG, 78532 Tuttlingen (DE)
(72) Erfinder: LINKE, Ralph, 78234 Engen (DE)
(74) Vertreter: Winter, Brandl - Partnerschaft mbB

(57) **Zusammenfassung**

Die vorliegende Offenbarung betrifft ein medizinisches Instrument (2) zum Positionieren einer Pedikelschraube (100) zur Wirbelmanipulation, mit einem Gehäuse (4) und einer an dem Gehäuse (4) aufgenommenen Koppeleinheit (6), die in einer distal-proximal-Richtung des Instruments (2) relativ zu dem Gehäuse (4) bewegbar ist und eine proximale Basis (8) sowie zwei sich von der Basis (8) distal wegerstreckende, einander gegenüberliegende Koppelarme (10) aufweist, welche jeweils eine Raststruktur (12) zum lösbaren Ineingriffbringen mit einer Gegenraststruktur der Pedikelschraube (100) aufweisen, wobei das Instrument eine Rampenstruktur (14, 30) aufweist, entlang der die Koppelarme (10) bei einer distalen Bewegung der Koppeleinheit (4) zum Lösen des Rasteingriffs in eine medial-lateral-Richtung des Instruments (2) verlagert werden.

## Beschreibung

### Technisches Gebiet

Die vorliegende Offenbarung betrifft ein medizinisches Instrument zum Positionieren einer Pedikelschraube zur Wirbelmanipulation.

### Hintergrund

Pedikelschrauben dienen prinzipiell zur dorsalen Stabilisierung der Wirbelsäule bei Frakturen, Tumoren, Entzündungen, Deformitäten und degenerativ bedingten Instabilitäten mittels transpedikulärer Verschraubung. Dabei werden Pedikelschrauben in den Pedikeln jeweils benachbarter Wirbel platziert, worauf eine winkelstabile Verbindung zwischen den jeweils axial übereinander sich anordnenden Pedikelschrauben und einem axial sich erstreckenden Längsträger oder Steg geschaffen wird. Die Pedikelschrauben und Längsträger bilden dabei ein Wirbel-Stabilisierungssystem.

Hierfür hat eine Pedikelschraube in der Regel einen axialen, schaftartigen Außengewindeabschnitt, an den sich schraubenkopfseitig eine Aufnahmehülse (eine sogenannte Tulpe) anschließt. Diese weist eine U-förmige Aufnahme und ein Innengewinde auf, wobei der Längsträger/Stab quer (zur Schaftachse bzw. Gewindeachse) in die Aufnahme eingelegt und mittels einer in das Innengewinde eingedrehten Schraube fixiert werden kann.

Grundsätzlich werden zwei Grundarten von Pedikelschrauben unterschieden, nämlich monoaxiale sowie polyaxiale Pedikelschrauben. Im Fall einer monoaxialen Pedikelschraube sind der Außengewindeabschnitt/Schaft und die Aufnahmehülse einstückig miteinander ausgebildet. Eine polyaxiale Pedikelschraube hat hingegen einen als separates Bauteil gefertigten Außengewindeschaft mit einem zumeist (halb-) kugelförmigen Schraubenkopf, der von der Aufnahmehülse nach Art eines Kugellagers umgriffen und im Übergangsbereich zwischen Kopf und Schaft hintergriffen wird. Auf diese Weise kann die Aufnahmehülse nach Versenken des Außengewindeschafts im Pedikelkanal eines Wirbels relativ dazu verschwenkt und/oder verdreht werden, um eine gewünschte Lage und Ausrichtung im Wesentlichen unabhängig zur Ausrichtung des Schafts zu erhalten. Anschließend wird die Aufnahmehülse durch die Schraube, die auch den Längsträger fixiert, (Ein-Schrauben-Prinzip) oder durch eine zusätzliche Schraube (Mehr-Schrauben-Prinzip) am Schraubenkopf lagefixiert.

Bei polyaxialen Pedikelschrauben ist es aufgrund der Beweglichkeit zwischen dem Außengewindeschaft mit Schraubenkopf/Schaftkopf und der Aufnahmehülse nicht möglich, von der Aufnahmehülse aus Kräfte in den Wirbel einzuleiten, um diesen beispielsweise manipulieren zu können. Dadurch, dass die finale Lagefixierung der Aufnahmehülse am Schraubenkopf unter anderem aufgrund von Selbsthemmung nicht ohne Weiteres lösbar ist, ist sie weniger geeignet, um als temporäre Verriegelung bei der Wirbeljustierung die Beweglichkeit zu dienen. Daher werden der Außengewindeschaft und die Aufnahmehülse der polyaxialen Pedikelschrauben, d.h. die Polyaxialität der Pedikelschrauben, zur Wirbelmanipulation temporär/provisorisch fest miteinander verbunden. Diese temporäre Fixierung kann durch das Aufbringen einer Anpresskraft auf einen Klemmstempel der Aufnahmehülse, etwa durch einen distal verlagerbaren Druckstößel eines beispielsweise aus der DE 10 2016 108 504 A1 bekannten, zur Wirbelmanipulation eingesetzten Instruments, oder durch Verriegeln eines beispielsweise aus der DE 10 2011 053 295 A1 bekannten Verriegelungsmechanismus an der Pedikelschraube selbst oder (in begrenztem Maße) durch das Einlegen des Längsträgers in die Aufnahmehülse beim Aufsetzen des zur Wirbelmanipulation eingesetzten Instruments erfolgen.

Um die Wirbel manipulieren zu können bzw. um Kräfte auf die Pedikelschraube (bzw. die Aufnahmehülse der Pedikelschraube) aufbringen zu können, ist es erforderlich, das Instrument mit der Pedikelschraube (lösbar) zu koppeln. Dazu weist das Instrument eine Koppeleinheit mit einer Raststruktur auf, die mit einer Gegenraststruktur an der Pedikelschraube (bzw. an der Aufnahmehülse der Pedikelschraube) in lösbaren Eingriff bringbar ist, so dass (in einem verbundenen Zustand) die Kräfte über das Instrument formschlüssig in die Pedikelschraube eingeleitet werden können.

Bei dem aus der DE 10 2016 108 504 A1 bekannten Instrument zur Wirbelmanipulation weist die Koppeleinheit zwei gegenüberliegende, sich distal von einer proximalen Basis aus wegerstreckende Koppelarme auf, in deren distalem Endbereich die Raststruktur ausgebildet ist. Dabei ist die Koppeleinheit relativ zu einer Gehäusehülse des Instruments in einer distal-proximal-Richtung des Instruments verlagerbar, wobei die Koppelarme beim Bewegen in die proximale Richtung entlang des Gehäuses derart (elastisch) verlagert werden, dass die Raststruktur in einen Rasteingriff mit der Pedikelschraube gepresst wird, so dass ein Lösen des Rasteingriffs in einer proximalen Position der Koppeleinheit nicht möglich ist. In einer distalen Position der Koppeleinheit kann der Rasteingriff zwischen der Koppeleinheit und der Pedikelschraube gelöst werden, jedoch ist aufgrund der selbsthemmenden Ausbildung des Rasteingriffs dafür ein zusätzliches Werkzeug (ein sogenanntes Removal-Instrument) erforderlich.

Weiter ist aus der WO 2020 / 201 376 A1 ein Instrument zur Wirbelmanipulation bekannt, bei dem die Raststruktur an einem distalen Ende von nach Art eines Hebelmechanismus schwenkbar gelagerten Rastarmen ausgebildet ist und durch Aufbringen einer an einem proximalen Ende der Rastarme (insbesondere über ein zusätzliches Werkzeug (ein sogenanntes Removal-Instrument)) eingeleiteten Kraft lösbar ist. Dabei ist es zur Gewährleistung der Montage erforderlich, dass die Rastarme mit Spiel gelagert sind, so dass sich die Raststruktur in Form von Haltenasen verhaken kann und somit ggf. nicht lösbar ist. Zudem ist es erforderlich, die Rastarmen mit einer verhältnismäßig großen Elastizität auszubilden, so dass bereits eine geringe äußere Druckkraft (etwa durch das Gewebe in situ, ausgelöst durch Kohäsionskräfte der Muskeln oder Steifigkeit der Knochen) ein Lösen/ein Entformen der Raststruktur verhindern kann.

Es ist also die Aufgabe der vorliegenden Offenbarung, die Nachteile aus dem Stand der Technik zu vermeiden oder wenigstens zu verringern. Insbesondere soll ein Instrument zur Wirbelmanipulation bereitgestellt werden, mit dem Kräfte in eine Pedikelschraube zuverlässig eingebracht werden können und das gleichzeitig einfach aufgebaut sowie zu bedienen ist. Insbesondere soll dabei ein Rasteingriff zwischen der Pedikelschraube und dem Instrument zuverlässig und einfach, vorzugsweise ohne zusätzliche Hilfsmittel, lösbar sein.

Die Aufgabe der vorliegenden Offenbarung wird durch ein medizinisches Instrument mit den Merkmalen des unabhängigen Patentanspruchs gelöst. Vorteilhafte Ausgestaltungen sind Gegenstand der Unteransprüche.

Demnach betrifft die vorliegende Offenbarung ein medizinisches Instrument zum Positionieren einer (vorzugsweise polyaxialen) Pedikelschraube zur Wirbelmanipulation. Das Instrument weist ein Gehäuse und eine an dem Gehäuse aufgenommenen Koppeleinheit zur Kopplung, d.h. zur fixierten/festen, aber lösbaren Verbindung, mit der Pedikelschraube auf. Die Koppeleinheit ist in einer distal-proximal-Richtung des Instruments relativ zu dem Gehäuse (translatorisch) bewegbar. Die distal-proximal-Richtung entspricht einer Richtung entlang einer Längsachse des Instruments und kann auch als eine Längsrichtung bzw. Axialrichtung bezeichnet werden. Die Koppeleinheit weist eine proximale, d.h. bedienerseitige, Basis sowie zwei sich von der Basis distal wegerstreckende, einander (insbesondere diametral) gegenüberliegende Koppelarme auf. Beispielsweise können die Koppelarme durch Kohäsion mit der Basis verbunden sein, beispielsweise Schweißen, Löten und/oder Verstiften. Die Koppelarme weisen jeweils eine Raststruktur zum lösbaren Ineingriffbringen/Koppeln mit einer Gegenraststruktur der Pedikelschraube auf. Das heißt, dass die Raststruktur und die Gegenraststruktur in einen Rasteingriff bringbar sind, in welchem sie eine formschlüssige, durch elastische Verformung der Raststruktur und/oder der Gegenraststruktur lösbare Schnappverbindung ausbilden.

Gemäß der vorliegenden Offenbarung weist das Instrument eine Rampenstruktur auf, entlang der die Koppelarme bei einer distalen Bewegung der Koppeleinheit relativ zu dem Gehäuse in eine medial-lateral-Richtung des Instruments zum Lösen des Rasteingriffs verlagert werden. Das heißt, dass die Koppelarme derart verlagert werden, dass die Raststruktur außer Eingriff mit der Gegenraststruktur gelangt bzw. gebracht wird oder bringbar ist. Die medial-lateral-Richtung entspricht einer Richtung quer, vorzugsweise senkrecht zu der distal-proximal-Richtung und kann auch als eine Radialrichtung bezeichnet werden. Das heißt, dass die Rampenstruktur eine Zwangsentformung des Rasteingriffs führt bzw. verursacht, wenn die Koppeleinheit relativ zu dem Gehäuse nach distal, d.h. nach vorne/in Richtung zur Pedikelschraube hin bzw. vom Bediener weg, verlagert wird. Mit anderen Worten wird die Raststruktur bzw. werden die Koppelarme in eine Rasteingriff-Entformungsrichtung verlagert.

Anders ausgedrückt ist die Raststruktur am freien Ende der Koppelarme ausgebildet, die aufgrund ihrer kragenden Ausbildung (nämlich von der Basis aus in die distale Richtung) (elastisch) aufeinander zu bzw. voneinander weg bewegbar/schwenkbar sind, um den Rasteingriff einzugehen/gebracht zu werden bzw. entformen/lösen zu können. Dabei weist das Instrument die Rampenstruktur auf, die derart ausgebildet und angeordnet ist, dass sie bei der distalen Bewegung der Koppeleinheit die Verlagerung der Koppelarme quer zu der distal-proximal-Richtung führt bzw. erzwingt. Mit anderen Worten wird durch die Rampenstruktur die distale Bewegung der Koppeleinheit mit der medial-lateralen Bewegung der Koppelarme gekoppelt, und zwar in die Richtung, in die der Rasteingriff lösbar ist.

Dies hat den Vorteil, dass das Instrument reproduzierbar und zuverlässig nur durch distales Verlagern der Koppeleinheit relativ zu dem Gehäuse und damit ohne weitere Zusatzinstrumente, sondern durch Zwangsentformung von der Pedikelschraube abnehmbar/abkoppelbar/lösbar ist.

Gemäß einer bevorzugten Ausführungsform können die Koppelarme jeweils dem anderen der Koppelarme zugewandte Innenflächen aufweisen und die Raststruktur an den Innenflächen ausgebildet sein, wobei die Rampenstruktur derart ausgebildet ist, dass die Koppelarme bei der distalen Bewegung der Koppeleinheit relativ zu dem Gehäuse in der medial-lateral-Richtung nach außen, d.h. (jeweils) lateral bzw. weiter von der Längsachse weg, verlagert werden (bzw. die Koppelarme bzw. die Raststruktur auseinander bewegt bzw. aufgespreizt werden). Mit anderen Worten ist die Pedikelschraube zwischen den Koppelarmen aufnehmbar und entsprechend der Rasteingriff durch (die Rampenstruktur geführtes) Auseinanderdrücken der Koppelarme lösbar. Anders ausgedrückt wird durch die Rampenstruktur die distale Bewegung der Koppeleinheit mit der lateralen Bewegung der Koppelarme gekoppelt. Das heißt, dass die Koppelarme bzw. die innenseitig ausgebildete Raststruktur in einer proximalen (End-)Position/Befestigungsposition/Rastposition näher zu der Längsachse des Instruments als in einer distalen (End-)Position/Freigabeposition angeordnet sind. Durch die Anordnung der Raststruktur an den Innenflächen kann die Gegenraststruktur an der Pedikelschraube in bevorzugter Weise außenseitig ausgebildet werden.

Gemäß einer bevorzugten Ausführungsform kann die Rampenstruktur durch eine gehäusefeste Schräge und/oder eine koppelarmfeste Schräge gebildet sein. Das heißt, dass die Rampenstruktur durch eine an dem Gehäuse ausgebildete Schräge und/oder durch eine an den Koppelarmen ausgebildete Schräge ausgebildet sein kann. Insbesondere kann die Schräge an einer Außenfläche des Gehäuses (in der medial-lateral-Richtung) bzw. an einer Innenfläche des jeweiligen Koppelarms (in der medial-lateral-Richtung) angeordnet sein. Dadurch ist die Führung der Koppelarme nach außen konstruktiv einfach realisierbar. Vorzugsweise kann die Rampenstruktur durch sowohl an dem Gehäuse als auch an den Koppelarmen, vorzugsweise korrespondierend ausgebildete Schrägen gebildet sein kann. So kann das (Auseinander-)Gleiten der Koppelarme bzw. die Zwangsentformung der Raststruktur besonders sanft geführt werden.

Gemäß einer bevorzugten Ausführungsform kann die Raststruktur an einem distalen Endbereich der Koppelarme ausgebildet sein und die Rampenstruktur (am Gehäuse und/oder am jeweiligen Koppelarm) proximal der Raststruktur angeordnet sein. Das heißt, dass die Raststruktur der Rampenstruktur vorauseilend angeordnet ist. So kann die Kopplung mit der Pedikelschraube besonders vorteilhaft umgesetzt werden.

Gemäß der bevorzugten Ausführungsform kann die Rampenstruktur direkt in der distal-proximal-Richtung benachbart zu der Raststruktur angeordnet sein, d.h. ebenfalls in dem distalen Endbereich der Koppelarme bzw. in einem distalen Endbereich des Gehäuses ausgebildet sein. Durch die Anordnung im Bereich der freikragenden Enden der Koppelarme kann gewährleistet werden, dass die Kraft an einer Stelle der Koppelarme eingeleitet wird, die besonders leicht elastisch verlagerbar ist.

Gemäß einer bevorzugten Ausführungsform kann die Rampenstruktur je Koppelarm zwei Rampenabschnitte aufweisen und die Raststruktur in einer Richtung, die quer, insbesondere senkrecht zu der medial-lateral-Richtung und quer, insbesondere senkrecht zu der distal-proximal-Richtung ist, zwischen den Rampenabschnitten angeordnet sein. Das heißt, dass die Rampenabschnitte beidseitig der Raststruktur angeordnet sind, wodurch vorteilhafterweise das Lösen des Rasteingriffs so geführt ist, dass ein Risiko für ein Verkanten der Rampenstruktur reduziert wird.

Gemäß einer bevorzugten Ausführungsform können die Koppelarme derart vorgespannt und/oder material-elastisch ausgebildet sein, dass der Rasteingriff entgegen einer Vorspannung und/oder entgegen einer (Eigen-) Elastizität der Koppelarme lösbar ist. Mit anderen Worten ist die Pedikelschraube im Rasteingriff spielfrei mit den Koppelarmen verspannt. Somit wird ein Risiko eines elastischen Verspannens mit der Pedikelschraube reduziert. Weiter wird durch die spielfreie Ausbildung sichergestellt, dass eine (Längs-)Achse der Pedikelschraube und eine (Längs-)Achse des Instruments nicht Verkippen, was zur Folge hätte, dass eine Schraube zur Fixierung der Pedikelschraube, wie eine Implantat-Madenschraube, nicht mehr axial fluchtend eingebracht würde und ein Innengewinde der Pedikelschraube beschädigt werden könnte, was sogar zu einem Versagen der Pedikelschraube während der Lebensdauer im Patienten führen könnte.

Gemäß einer bevorzugten Ausführungsform kann das Gehäuse je Koppelarm einen sich parallel zu der distal-proximal-Richtung erstreckenden, außenseitig (d.h. quer zu der distal-proximal-Richtung) offenen Kanal aufweisen, in dem jeweils einer der Koppelarme aufgenommen ist. Das heißt, dass das Gehäuse zur Aufnahme der Koppelarme eine Längsausnehmung aufweist. Mit anderen Worten ist der Koppelarm in dem Kanal lateral/nach außen verlagerbar bzw. in seiner lateralen Verlagerbarkeit nicht durch das Gehäuse beschränkt. Somit wird gewährleistet, dass die Koppelarme aufgespreizt werden können.

Gemäß einer bevorzugten Ausführungsform kann das Gehäuse einen Steg (bzw. einen Steg je Koppelarm) aufweisen. Der Steg kann vorzugsweise (direkt) proximal zu dem Kanal angeordnet sein. Der Steg ist in einem proximalen Endbereich der Koppelarme angeordnet. Das heißt, dass der Steg distal der Basis der Koppeleinheit angeordnet ist. Der Steg übergreift den Koppelarm bzw. die Koppelarme außenseitig (d.h. lateral bzw. quer zu der distal-proximal-Richtung außerhalb der Koppelarme). Das heißt, dass der Steg die laterale Verlagerbarkeit/das Aufspreizen der Koppelarme begrenzt. Je weiter die Koppeleinheit distal verlagert werden, desto weiter weg ist der Steg von der Raststruktur entfernt, so dass die Begrenzung der lateralen Verlagerbarkeit mit der distalen Bewegung der Koppeleinheit reduziert wird.

Gemäß der bevorzugten Ausführungsform kann der Steg einen Anschlag zur Begrenzung der distalen Bewegung der Koppeleinheit bilden. Das heißt, dass beispielsweise die Basis der Koppeleinheit in einer distalen Endposition der Koppeleinheit an dem Steg anliegt. Somit kann verhindert werden, dass die Koppeleinheit zu weit in die distale Richtung verlagert wird.

Gemäß einer bevorzugten Ausführungsform kann das Instrument eine (manuell betätigbare) Stellmutter aufweisen, die mit dem Gehäuse in Gewindeeingriff steht und in der distal-proximal-Richtung mit der Koppeleinheit verbunden ist. Eine Gewindeachse des Gewindeeingriffs ist vorzugsweise parallel zur distal-proximal-Richtung. Das heißt, dass die Koppeleinheit und das Gehäuse über ein Gewinde (an der Stellmutter) miteinander in Eingriff stehen und durch gegenseitiges Verdrehen in der distal-proximal-Richtung positionierbar sind. Die Stellmutter ist vorzugsweise proximal der Koppelarme angeordnet. Somit kann durch Verdrehen der Stellmutter relativ zu dem Gehäuse die Koppeleinheit in der distal-proximal-Richtung relativ zu dem Gehäuse positioniert werden. Dabei kann die Stellmutter vorzugsweise als ein von der Basis separates Bauteil ausgebildet, das beispielsweise über einen PEEK-Ring und einen Wellensicherungsring mit der Basis verbunden ist. Der Gewindeeingriff kann vorzugsweise durch ein metrisches Feingewinde gebildet sein. Der Gewindeeingriff kann, beispielsweise in Abhängigkeit einer Anwenderpräferenz, als ein Linksgewinde oder ein Rechtsgewinde ausgebildet sein.

Gemäß einer bevorzugten Ausführungsform kann die Koppeleinheit zwischen einer distalen Endposition, in der der Rasteingriff vorzugsweise lösbar oder gelöst ist, und einer proximalen Endposition, in der der Rasteingriff vorzugsweise nicht lösbar oder nicht gelöst ist, verlagerbar sein. Vorzugsweise kann der Rasteingriff ausschließlich in der distalen Endposition lösbar sein.

Gemäß der bevorzugten Ausführungsform kann der Gewindeeingriff derart ausgebildet sein, dass ein Verdrehen der Stellmutter um maximal 200°, vorzugsweise um maximal 150°, insbesondere um maximal 90 bis 100°, die Koppeleinheit zwischen der distalen Endposition und der proximalen Endposition bewegt. Das heißt, dass eine Gewindesteigung derart gewählt ist, dass eine Verriegelung (von Endposition zu Endposition) durch maximal drei Drehbewegungen eines Handgelenks, insbesondere um maximal ein bis zwei Drehbewegungen erfolgen kann.

Die vorliegende Offenbarung betrifft auch einen Bausatz aus einem beschriebenen Instrument und einer Pedikelschraube. Die (vorzugsweise polyaxiale) Pedikelschraube weist einen schaftartigen Außengewindeabschnitt mit einem vorzugsweise (halb-)kugelförmigen Schraubenkopf und eine sich schraubenkopfseitig an den Außengewindeabschnitt anschließende Aufnahmehülse auf. Zudem weist die Aufnahmehülse vorzugsweise auf einer Außenumfangsfläche eine Gegenraststruktur auf, in die die Koppeleinheit, insbesondere die Raststruktur an den Koppelarmen, im Rasteingriff/in dem gekoppelten Zustand eingreift.

Mit anderen Worten betrifft die vorliegende Offenbarung einen optimierten Kopplungsmechanismus eines verschraubbaren Wirbel(säulen)manipulators, bei dem der Verbindungsmechanismus ähnlich zu der DE 10 2016 108 504 A1 ausgeführt ist, aber das Festziehen der Haltenasen mit dem Gewinde das Spiel an der Verbindungsstelle auf 0 reduziert. Zudem ist neu, dass im Arbeitsende des Wirbelmanipulators eine Rampe und an der Haltenase eine korrespondierende Schräge angebracht wird, so dass im Falle eines Öffnens aus der axialen (d.h. distal-proximalen, insbesondere distalen) Bewegung eine Zwangsentformung senkrecht zur axiale Achse realisiert ist. Die Elastizität der Haltenasen erleichtert die Zwangsentformung. Insbesondere kann eine Rampe am Arbeitsende des Wirbelmanipulators und eine Rampe an der Haltenase ausgebildet sein, um die axiale Schraubenbewegung in einer Bewegung senkrecht zur Achse umzusetzen. Zudem kann eine Elastizität der Haltenasen eine Zwangsentformung vereinfachen. Weiter kann eine Steigung des Gewindes (beispielsweise ausgeführt als metrisches Links-Feingewinde) so gewählt sein, dass die Verriegelung durch eine Drehbewegung des Handgelenks (maximal 3 Drehbewegungen des Handgelenks) erfolgen kann (Drehbewegung der Drehhülse bzw. Stellmutter um 90°, bzw. zwischen 0° und 200°). Ferner kann ein Aufsetzen und Abnehmen des Instruments ohne Zuhilfenahme weiterer Instrumente möglich gemacht werden. Insbesondere ist die Raststruktur an den Koppelarmen ausgebildet, welche im Kanal des Gehäuses gleiten. Eine proximale Basis der Koppeleinheit wurde in die die Bauteile Ring und Stellmutter geteilt, wobei der Ring und die Stellmutter über einen PEEK-Ring und einen Wellensicherungsring verbunden sein können. Eine Innenbohrung der Stellmutter ist ein Transportgewinde, welches als Links- oder Rechtsgewinde ausgeführt werden kann (Abhängig von der Präferenz der Anwender). Die Koppelarme und der Ring werden durch Kohäsion fester Körper verbunden (etwa durch Schweißen, Löten, Verstiften). Wird die Stellmutter gedreht, bewegt sich die Baugruppe Koppelarme und Ring im Gehäuse in proximal-distaler Richtung. Am distalen Ende des Kanals befindet sich eine Rampe auf der die korrespondierenden Rampen der Koppelarme gleiten, sodass die Raststruktur außer Eingriff gerät, weil sie sich in medial-lateraler Richtung bewegt, wenn die Baugruppe sich nach distal bewegt. Bewegt sich die Baugruppe nach proximal, wird die Raststruktur aufgrund der Eigenelastizität der Koppelarme in die Gegenraststruktur gezogen, die Implantat-Schraube wird im Kopplungsmechanismus spielfrei verspannt. So ist eine Wirbelkörper-Manipulation möglich, ohne dass Teile des Instruments sich im Kopplungsmechanismus gegeneinander elastisch verspannen. Zudem ist das Problem, dass das Instrument nicht abnehmbar ist, gelöst. Die Koppelarme können mit einer T-Nut oder ohne eine T-Nut ausgebildet sein.

### Kurzbeschreibung der Figuren

Fig. 1 ist eine perspektivische Darstellung eines medizinischen Instruments gemäß einer ersten Ausführungsform der vorliegenden Offenbarung;
Fig. 2 ist eine perspektivische Darstellung einer Koppeleinheit des Instruments;
Fig. 3 ist ein vergrößerter Ausschnitt eines distalen Endbereichs der Koppeleinheit;
Fig. 4 ist eine perspektivische Darstellung eines Gehäuses des Instruments;
Fig. 5 ist ein vergrößerter Ausschnitt eines distalen Endbereichs des Gehäuses;
Fig. 6 ist eine perspektivische Darstellung des medizinischen Instruments gemäß einer zweiten Ausführungsform der vorliegenden Offenbarung; und
Fig. 7 ist eine beispielhafte Darstellung einer Pedikelschraube.

### Beschreibung von bevorzugten Ausführungsformen

Nachstehend werden bevorzugte Ausführungsformen der vorliegenden Offenbarung auf der Basis der zugehörigen Figuren beschrieben.

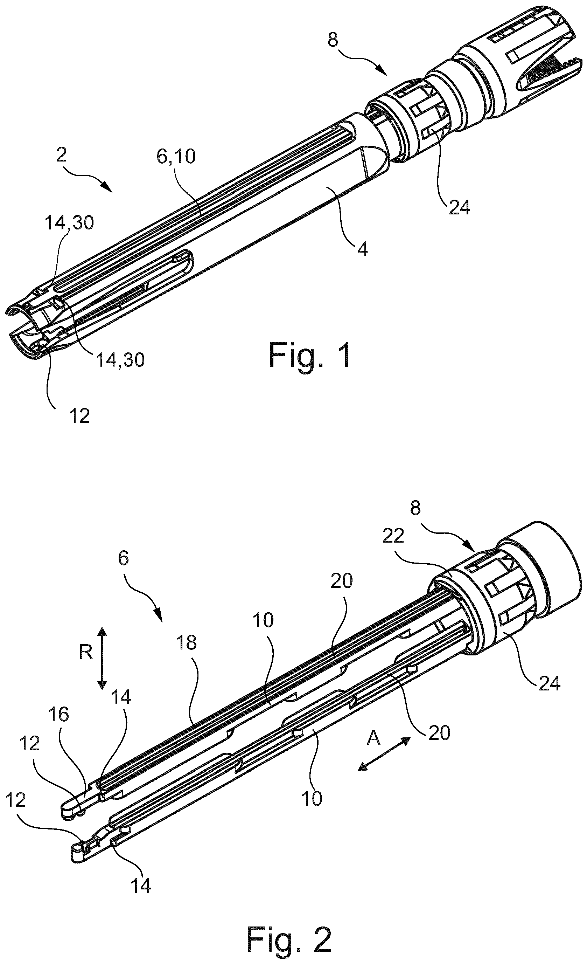

Fign. 1 bis 5 zeigen eine erste Ausführungsform eines medizinisches Instruments 2 gemäß der vorliegenden Offenbarung.

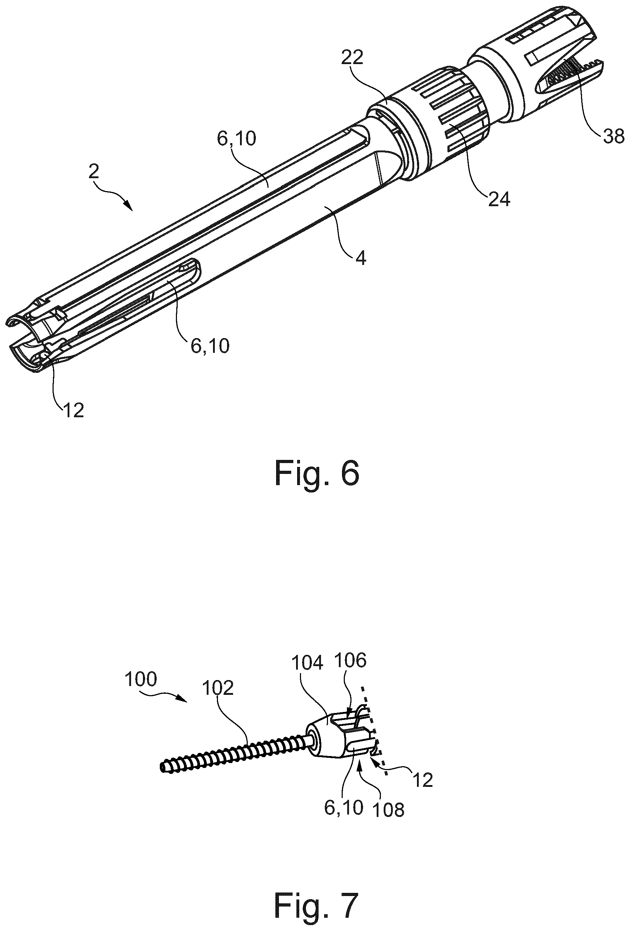

Das Instrument 2 dient zum Positionieren einer vorzugsweise polyaxialen Pedikelschrauben 100 (vgl. Fig. 7) zur Wirbelmanipulation.

Im Folgenden wird eine distal-proximal-Richtung des Instruments 2, die sich im Wesentlichen entlang einer Längsachse des Instruments 2 erstreckt, auch als eine Axialrichtung A oder Längsrichtung bezeichnet. Ein proximales (bedienerseitiges/patientenfernes) Ende ist in Axialrichtung hinten, d.h. an einem Bedienende, angeordnet. Ein distales (bedienerabgewandtes/patientennahes) Ende ist in Axialrichtung vorne, d.h. an einer Instrumentenspitze/einem Arbeitsende angeordnet.

Im Folgenden wird eine medial-lateral-Richtung des Instruments 2, die sich im Wesentlichen quer, vorzugsweise senkrecht zu der Längsachse des Instruments 2 erstreckt und die einer Richtung quer, vorzugsweise senkrecht zu der distal-proximal-Richtung entspricht, auch als eine Radialrichtung R bezeichnet. Unter lateral wird eine radial außen/außenseitig befindliche Anordnung/Erstreckung verstanden und unter medial wird eine radial innen/innenseitig befindliche Anordnung/Erstreckung verstanden.

Das Instrument 2 weist ein Gehäuse 4 und eine an dem Gehäuse 4 aufgenommene Koppeleinheit 6 auf. Die Koppeleinheit 6 dient zur Kopplung, zur fixierten/festen, aber insbesondere lösbaren Verbindung mit der Pedikelschraube 100. Die Koppeleinheit 6 ist in der distal-proximal-Richtung relativ zu dem Gehäuse 4 (translatorisch) bewegbar. Das heißt, dass die Koppeleinheit 6 axialverschieblich in dem Gehäuse 4 aufgenommen ist. Die Koppeleinheit 6 ist zwischen einer distalen Endposition und einer proximalen Endposition relativ zu dem Gehäuse 4 verlagerbar sein.

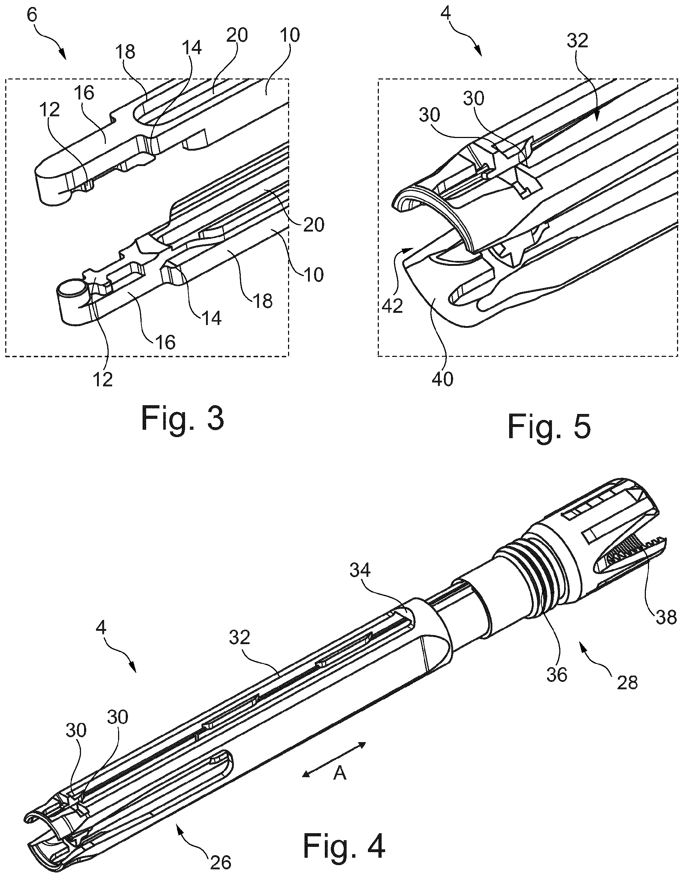

Die Koppeleinheit 6 (vgl. Fign. 2 und 3) weist eine proximale Basis 8 sowie zwei sich von der Basis 8 distal wegerstreckende Koppelarme 10 auf. Die Basis 8 und die Koppelarme 10 sind insbesondere fest miteinander verbunden. Beispielsweise sind die Basis 8 und die Koppelarme 10 durch Schweißen, Löten und/oder Verstiften verbunden.

Die Koppelarme 10 sind einander, insbesondere diametral gegenüberliegend angeordnet. Längsachsen der jeweiligen Koppelarme 10 erstrecken sich im Wesentlichen axial und sind radial beabstandet und parallel zueinander angeordnet. Die Koppelarme 10 weisen jeweils dem anderen der Koppelarme 10 zugewandte Innenflächen und jeweils von dem anderen der Koppelarme 10 abgewandte Außenflächen auf. Die Koppelarme 10 weisen vorzugsweise eine im Wesentlichen plattenförmige Ausbildung, d.h. im Querschnitt rechteckige Ausbildung, auf. Die Innenflächen und/oder die Außenflächen sind insbesondere senkrecht zur Radialrichtung ausgerichtet. Die Koppelarme 10 bilden insbesondere jeweils ein freies Ende der Koppeleinheit 6. Das heißt, dass die Koppelarme 10 nur an ihrem proximalen Ende über die Basis 8 miteinander verbunden sind.

Die Koppelarme 10 weisen jeweils eine Raststruktur 12 zum lösbaren Ineingriffbringen/Koppeln mit einer Gegenraststruktur 108 der Pedikelschraube 100 auf. Das heißt, dass die Raststruktur 12 und die Gegenraststruktur 108 in einen Rasteingriff bringbar sind, in welchem sie eine formschlüssige, durch elastische Verformung der Raststruktur 12 und/oder der Gegenraststruktur 108 lösbare Schnappverbindung ausbilden. Die Raststruktur 12 ist vorzugsweise an einem distalen Endbereich der Koppelarme 10, d.h. an einem freien Ende der Koppelarme 10, ausgebildet. Vorzugsweise ist die Raststruktur 12 an den Innenflächen der Koppelarme 10 ausgebildet. Das heißt, dass die Gegenraststruktur 108 entsprechend vorzugsweise an einer Außenfläche der Pedikelschraube 100 ausgebildet ist.

Vorzugsweise können die Koppelarme 10 derart vorgespannt und/oder material-elastisch ausgebildet sein, dass der Rasteingriff entgegen einer Vorspannung und/oder entgegen einer (Eigen-) Elastizität der Koppelarme 10 lösbar ist. Insbesondere kann die Koppeleinheit 10 mit der Pedikelschraube 100 im Rasteingriff spielfrei verspannt sein.

In der distalen Endposition der Koppeleinheit 6 ist der Rasteingriff vorzugsweise lösbar bzw. gelöst, und in der proximalen Endposition der Koppeleinheit 6ist der Rasteingriff vorzugsweise nicht lösbar bzw. nicht gelöst ist. Vorzugsweise kann der Rasteingriff ausschließlich in der distalen Endposition der Koppeleinheit 6 lösbar sein.

Zudem weist das Instrument 2 eine Rampenstruktur auf. Die Rampenstruktur ist derart ausgebildet, dass die Koppelarme 10 bei einer distalen Bewegung der Koppeleinheit 6 relativ zu dem Gehäuse 4 entlang der Rampenstruktur in die medial-lateral-Richtung zum Lösen des Rasteingriffs, d.h. zum außer Eingriff mit der Gegenraststruktur 108 bringen, verlagert werden. Die Rampenstruktur ist vorzugsweise so ausgebildet, dass die Koppelarme 10 durch die distale Bewegung der Koppeleinheit 6 lateral, d.h. nach außen bewegt werden bzw. auseinandergespreizt werden.

Die Rampenstruktur kann vorzugsweise proximal der Raststruktur 12 angeordnet sein. Insbesondere kann die Rampenstruktur direkt in der distal-proximal-Richtung benachbart zu der Raststruktur 12 angeordnet sein.

Die Rampenstruktur kann gehäuseseitig, d.h. an dem Gehäuse 4, und/oder koppelarmseitig, d.h. an den Koppelarmen 6, ausgebildet sein.

In der dargestellten Ausführungsform weisen die Koppelarme 10 jeweils eine Schräge 14 auf, die die Rampenstruktur ausbilden. Die Schräge 14 erstreckt sich geneigt zur Radialrichtung und geneigt zur Axialrichtung. An der Schräge 14 erstreckt sich der jeweilige Koppelarm 10 distal und lateral bzw. proximal und medial. Die Schräge 14 ist in dem distalen Endbereich der Koppelarme 10 angeordnet. Die Schräge 14 ist auf einer Innenseite der Koppelarme 10 angeordnet.

Die Koppelarme 10 weisen jeweils einen Rastabschnitt 16 und einen Zungenabschnitt 18 auf, der vorzugsweise in Breitenrichtung des Koppelarms 10 größer als der Rastabschnitt 16 ist und sich insbesondere zu beiden Seiten in Breitenrichtung des Rastabschnitts 16 schulterartig vergrößert. Der Zungenabschnitt 18 geht distal in den Rastabschnitt 16 über und proximal in die Basis 8 über. Im Bereich des Zungenabschnitts 18 ist eine Längsnut 20 ausgebildet. Die Längsnut 20 ist in der dargestellten Ausführungsform im Querschnitt T-förmig ausgebildet. Die Schräge 14 wird durch zwei Rampenabschnitte im Bereich des schulterartigen Übergangs zwischen dem Rastabschnitt 16 und dem Zungenabschnitt 18 gebildet. Die Raststruktur ist insbesondere mittig zwischen den beiden Rampenabschnitten angeordnet. An die beiden Rampenabschnitte schließt sich in proximaler Richtung ein ebener Auflageabschnitt an, der sich senkrecht zu der Radialrichtung erstreckt.

Weiter weist die Koppeleinheit 6 einen Ring 22 auf, der die Basis 8 (mit-)ausbildet. Die Koppelarme 10 sind fest mit dem Ring 22 verbunden. Der Ring 22 umgreift das Gehäuse 4, d.h. umgibt das Gehäuse 4 radial außerhalb.

Zudem weist das Instrument die Stellmutter 24 auf. Vorzugsweise kann die Stellmutter 24 manuell betätigbar sein. Die Stellmutter 24 steht mit dem Gehäuse 4 in Gewindeeingriff. Eine Gewindeachse des Gewindeeingriffs ist vorzugsweise parallel zur distal-proximal-Richtung. Insbesondere kann die Stellmutter 24 ein Innengewinde aufweisen, das den Gewindeeingriff bildet. Der Gewindeeingriff kann vorzugsweise durch ein metrisches Feingewinde gebildet sein. Der Gewindeeingriff kann, beispielsweise in Abhängigkeit einer Anwenderpräferenz, als ein Linksgewinde oder ein Rechtsgewinde ausgebildet sein. Die Stellmutter 24 ist in der distal-proximal-Richtung mit der Koppeleinheit (fest) verbunden. Die Stellmutter 24 ist vorzugsweise proximal der Koppelarme 10 angeordnet.

Die Koppeleinheit 6 und das Gehäuse 4 sind über die Stellmutter 24 bzw. den Gewindeeingriff zwischen der Stellmutter 24 und dem Gehäuse 4 in der distal-proximal-Richtung positionierbar. Durch Verdrehen der Stellmutter 24 relativ zu dem Gehäuse 4 verlagert sich die Koppeleinheit 6 in der distal-proximal-Richtung relativ zu dem Gehäuse 4.

Die Stellmutter 24 kann als ein von der Basis 8 bzw. dem Ring 22 separates Bauteil ausgebildet sein. Insbesondere kann die Stellmutter 24 über PEEK-Ring und einen Wellensicherungsring mit der Basis 8 bzw. dem Ring 22 verbunden sein. Alternativ kann die Stellmutter 24 auch integral mit der Basis 8 ausgebildet sein.

Vorzugsweise kann der Gewindeeingriff zwischen der Stellmutter 24 und dem Gehäuse 4 derart ausgebildet sein, dass ein Verdrehen der Stellmutter um maximal 200°, vorzugsweise um maximal 150°, insbesondere um maximal 90 bis 100°, die Koppeleinheit zwischen der distalen Endposition und der proximalen Endposition bewegt bzw. zwischen einer Rasteingriffsposition und einer Entkopplungsposition.

Das Gehäuse 4 (vgl. Fign. 4 und 5) ist hülsenartig mit einem zentralen Hohlraum entlang einer Längsachse des Gehäuses 4 ausgebildet. In einem distalen Abschnitt 26 des Gehäuses 4, in dem die Koppelarme 10 aufgenommen sind, ist das Gehäuse 4 im Querschnitt im Wesentlichen rechteckig bzw. quadratisch ausgebildet. In einem proximalen Abschnitt 28 des Gehäuses 4, in dem die Basis 8 ausgenommen ist, ist das Gehäuses 4 im Querschnitt im Wesentlichen rund bzw. kreisförmig ausgebildet.

In der dargestellten Ausführungsform weist das Gehäuse 4 (je Koppelarm 10) eine Schräge 30 auf, die die Rampenstruktur ausbildet. Die Schräge 30 erstreckt sich geneigt zur Radialrichtung und geneigt zur Axialrichtung. An der Schräge 30 erstreckt das Gehäuse 4 distal und lateral bzw. proximal und medial. Die Schräge 30 ist in einem distalen Endbereich des Gehäuses 4 angeordnet. Die Schräge 30 ist auf einer Außenseite des Gehäuses 4 angeordnet.

Die Schräge 14 und die Schräge 30 können insbesondere korrespondierend ausgebildet sein. Das heißt, dass die Rampenstruktur insbesondere sowohl an dem Gehäuse 4 als auch an den Koppelarmen 10 ausgebildet ist und die Schrägen 14, 30 zusammenwirken, um die Koppelarme 10 beim distalen Bewegen der Koppeleinheit 6 zum Gehäuse 4 nach außen zu drücken/verlagern.

Vorzugsweise kann das Gehäuse 4 je Koppelarm 10 einen sich parallel zu der distal-proximal-Richtung erstreckenden Kanal 32 aufweisen. Der Kanal 32 ist als eine Längsausnehmung und vorzugsweise außenseitig offen ausgebildet. In dem Kanal 32 ist jeweils einer der Koppelarme 10 aufgenommen. Eine Form des Kanals 32 entspricht im Längsschnitt im Wesentlichen einer Form des Koppelarms 10. Insbesondere weist der Kanal 32 einen schulterartigen Übergang auf und ist distal (im Bereich des Rastabschnitts 16) schmaler als proximal (im Bereich des Zungenabschnitts 18) ausgebildet. Vorzugsweise wird die Schräge 30 wird durch zwei Rampenabschnitte, insbesondere im Bereich des schulterartigen Übergangs, gebildet.

Vorzugsweise kann das Gehäuse einen Steg 34 (bzw. einen Steg 34 je Koppelarm 10) aufweisen. Der Steg 34 kann vorzugsweise (direkt) proximal zu dem Kanal 32 angeordnet sein. Der Steg 34 ist in einem proximalen Endbereich der Koppelarme 10 bzw. distal der Basis 8 der Koppeleinheit 6 angeordnet. Der Steg 34 übergreift den jeweiligen Koppelarm 10 außenseitig. Der Steg 34 bildet einen (distalen) Axialanschlag für die Koppeleinheit 6 und/oder einen (außenseitigen) Radialanschlag für den jeweiligen Koppelarm 10 aus.

Vorzugsweise weist das Gehäuse 4 in seinem proximalen Abschnitt 28 ein Außengewinde 36 auf, das mit der Stellmutter 24 den Gewindeeingriff bildet. Das Außengewinde 36 kann durch ein von dem die Kanäle 32 ausbildenden Teil separates Bauteil oder integral damit ausgebildet sein.

In der dargestellten Ausführungsform weist das Gehäuse 4 an seinem proximalen Ende eine Coupler-Einheit 38 auf. Die Coupler-Einheit 38 weist ein Innengewinde auf und ist zur Montage (von dem distalen Abschnitt 26 des Gehäuses 4) abnehmbar. Das Außengewinde 36 kann an der Coupler-Einheit 38 ausgebildet sein.

In der dargestellten Ausführungsform weist das Gehäuse 4 an seinem distalen Ende eine durch zwei diametral gegenüberliegende, im Querschnitt kreisbogenförmige Gehäuseabschnitte 40 gebildete kreisförmige Gehäuseöffnung 42 auf. In der Gehäuseöffnung 42 kann die Pedikelschraube 100 bzw. die Aufnahmehülse 104 der Pedikelschraube 100 aufgenommen werden.

Fig. 6 zeigt eine zweite Ausführungsform des medizinisches Instruments 2 gemäß der vorliegenden Offenbarung. Die zweite Ausführungsform entspricht im Wesentlichen der ersten Ausführungsform, wobei die Koppelarme 10 ohne T-Nut ausgebildet sind.

Fig. 7 ist eine beispielhafte Darstellung einer Pedikelschraube 100. Die Pedikelschraube 100 weist einen schaftartigen Außengewindeabschnitt 102 mit einem vorzugsweise (halb-)kugelförmigen Schraubenkopf und eine sich schraubenkopfseitig an den Außengewindeabschnitt 102 anschließende Aufnahmehülse 104 auf. Die Aufnahmehülse 104 wird auch als eine Tulpe bezeichnet. Die Pedikelschraube 100 ist als eine polyaxiale Pedikelschraube ausgebildet, bei der die Aufnahmehülse 104 den Schraubenkopf nach Art eines Kugellagers umgreift und somit verdrehbar und/oder verschwenkbar aufnimmt. Die Aufnahmehülse 104 weist eine U-förmige Aufnahme 106 auf, die durch zwei diametral gegenüberliegende Längsschlitze gebildet wird und zur Aufnahme eines Längsträgers/Stabs quer Gewindeachse/Schaftachse dient. Zudem weist die Aufnahmehülse 104 ein Innengewinde auf, in das eine Schraube zur Lagefixierung des Längsträgers eingeschraubt werden kann. Auf einer Außenumfangsfläche weist die Aufnahmehülse eine Gegenraststruktur 108 auf, in die die Koppeleinheit 6, insbesondere die Raststruktur 12 an den Koppelarmen 10, in dem gekoppelten Zustand eingreift und den Rasteingriff bildet.

## Patentansprüche

1. Medizinisches Instrument (2) zum Positionieren einer Pedikelschraube (100) zur Wirbelmanipulation, mit
einem Gehäuse (4) und einer an dem Gehäuse (4) aufgenommenen Koppeleinheit (6), die in einer distal-proximal-Richtung des Instruments (2) relativ zu dem Gehäuse (4) bewegbar ist und eine proximale Basis (8) sowie zwei sich von der Basis (8) distal wegerstreckende, einander gegenüberliegende Koppelarme (10) aufweist, welche jeweils eine Raststruktur (12) zum lösbaren Ineingriffbringen mit einer Gegenraststruktur der Pedikelschraube (100) aufweisen, **gekennzeichnet durch** eine Rampenstruktur (14, 30), entlang der die Koppelarme (10) bei einer distalen Bewegung der Koppeleinheit (4) zum Lösen des Rasteingriffs in eine medial-lateral-Richtung des Instruments (2) verlagert werden.

2. Instrument (2) nach Anspruch 1, **dadurch gekennzeichnet, dass** die Koppelarme (10) jeweils dem anderen der zwei Koppelarme (10) zugewandte Innenflächen aufweisen und die Raststruktur (12) an den Innenflächen ausgebildet ist, wobei die Rampenstruktur (14, 30) derart ausgebildet ist, dass die Koppelarme (10) bei der distalen Bewegung der Koppeleinheit (6) in der medial-lateral-Richtung nach außen verlagert werden.

3. Instrument (2) nach Anspruch 1 oder 2, **dadurch gekennzeichnet, dass** die Rampenstruktur (14, 30) durch eine gehäusefeste Schräge (30) und/oder eine koppelarmfeste Schräge (14) gebildet ist.

4. Instrument (2) nach einem der Ansprüche 1 bis 3, **dadurch gekennzeichnet, dass** die Raststruktur (12) an einem distalen Endbereich der Koppelarme (10) ausgebildet ist und die Rampenstruktur (14, 30) proximal der Raststruktur (12) angeordnet ist.

5. Instrument (2) nach einem der Ansprüche 1 bis 4, **dadurch gekennzeichnet, dass** die Rampenstruktur (14, 30) je Koppelarm (10) zwei Rampenabschnitte aufweist und die Raststruktur (12) in einer Richtung, die quer zu der medial-lateral-Richtung und quer zu der distal-proximal-Richtung ist, zwischen den Rampenabschnitten angeordnet ist.

6. Instrument (2) nach einem der Ansprüche 1 bis 5, **dadurch gekennzeichnet, dass** die Koppelarme (10) derart vorgespannt und/oder material-elastisch ausgebildet sind, dass der Rasteingriff entgegen einer Vorspannung und/oder entgegen einer Elastizität der Koppelarme (10) lösbar ist.

7. Instrument (2) nach einem der Ansprüche 1 bis 6, **dadurch gekennzeichnet, dass** das Gehäuse (4) je Koppelarm (10) einen sich parallel zu der distal-proximal-Richtung erstreckenden, außenseitig offenen Kanal (32) aufweist, in dem jeweils einer der Koppelarme (10) aufgenommen ist.

8. Instrument (2) nach einem der Ansprüche 1 bis 7, **dadurch gekennzeichnet, dass** das Gehäuse (4) einen Steg (34) aufweist, der in einem proximalen Endbereich der Koppelarme (10) angeordnet ist und die Koppelarme (10) außenseitig übergreift.

9. Instrument (2) nach einem der Ansprüche 1 bis 8, **gekennzeichnet durch** eine Stellmutter (24), die mit dem Gehäuse (4) in Gewindeeingriff steht und in der distal-proximal-Richtung mit der Koppeleinheit (6) verbunden ist.

10. Instrument (2) nach Anspruch 9, **dadurch gekennzeichnet, dass** die Koppeleinheit (6) zwischen einer distalen Endposition, in der der Rasteingriff lösbar oder gelöst ist, und einer proximalen Endposition, in der der Rasteingriff nicht lösbar oder nicht gelöst ist, verlagerbar ist und der Gewindeeingriff derart ausgebildet ist, dass ein Verdrehen der Stellmutter (24) um maximal 200° die Koppeleinheit (6) zwischen der distalen Endposition und der proximalen Endposition bewegt.
